# EUROPÄISCHE PATENTSCHRIFT

(11) **EP 0 730 569 B1**
(45) Veröffentlichungstag und Bekanntmachung des Hinweises auf die Patenterteilung: **26.08.1998**
(21) Anmeldenummer: 95900675.0
(22) Anmeldetag: 08.11.1994
(51) Int. Cl.: C07C 69/54, C09K 19/38, C07C 43/215, G03G 5/07

(54) **NEUE TRIPHENYLENVERBINDUNGEN UND VERFAHREN ZUR HERSTELLUNG VON DISKOTISCH FLÜSSIGKRISTALLINEN VERNETZTEN POLYMEREN**
NOVEL TRIPHENYLENE COMPOUNDS AND PROCESS FOR PRODUCING DISCOIDALLY LIQUID CRYSTALLINE CROSS-LINKED POLYMERS
NOUVEAUX COMPOSES DU TRIPHENYLENE ET PROCEDE DE PREPARATION DE POLYMERES RETICULES SOUS FORME DE CRISTAUX LIQUIDES DISCOIDES

(30) Priorität: 22.11.1993 DE 4339711
(43) Veröffentlichungstag der Anmeldung: 11.09.1996
(73) Patentinhaber: BASF AKTIENGESELLSCHAFT, 67056 Ludwigshafen (DE)
(72) Erfinder: HAEUSSLING, Lukas, D-55452 Laubenheim (DE); FUNHOFF, Dirk, D-69126 Heidelberg (DE); SIEMENSMEYER, Karl, D-67227 Frankenthal (DE); ETZBACH, Karl-Heinz, D-67227 Frankenthal (DE); CLOSS, Friedrich, D-67227 Frankenthal (DE); RINGSDORF, Helmut, D-55124 Mainz (DE); SCHUHMACHER, Peter, D-55127 Mainz (DE)
(86) Internationale Anmeldenummer: EP9403649
(87) Internationale Veröffentlichungsnummer: WO9514652

(56) Entgegenhaltungen:
- EP-A- 0 385 329
- DE-A- 3 346 980
- DE-A- 3 827 046
- US-A- 4 865 762
- TETRAHEDRON (TETRAB,00404020);91; VOL.47 (4-5); PP.791-8, UNIV. RENNES I;LAB. ELECTROCHIM. ORG.; RENNES; 35042; FR. (FR) Chapuzet J M et al 'The anodic trimerization of aromatic orthodiethers: new developments'
- J. AM. CHEM. SOC. (JACSAT,00027863);91; VOL.113 (23); PP.8577-83, UNIV. MASSACHUSETTS;DEP. CHEM.; AMHERST; 01003; MA; USA (US) Collard D M et al 'Structure-property relationships for the thermal phase behavior of discotic liquid crystals: the effect of branching and unsaturation in the side chains of disklike molecules'
- POLYM. PREPR. (AM. CHEM. SOC., DIV. POLYM. CHEM.) (ACPPAY,00323934);92; VOL.33 (1); PP.1048-9, KENT STATE UNIV.;LIQ. CRYS. INST.; KENT; 44242; OH; USA (US) Shenouda L G et al 'Synthesis and properties of diacetylenic liquid crystals'
- LIQ. CRYST. ORDERED FLUIDS (LCOFDL,01465597);84; VOL.4,; PP.57-74, THOMSON-CSF;LAB. CENT. RECH.; ORSAY; 91401; FR. (FR) Le Barny P et al 'Some new thermotropic discogens'
- MOL. CRYST. LIQ. CRYST. SCI. TECHNOL., SECT. A (MCLCE9,1058725X);94; VOL.238,; PP.39-45, KUMAMOTO UNIV.;DEP. APPLIED CHEM.; KUMAMOTO; 860; JAPAN (JP) Kurihara S et al 'Intra- and intermolecular photodimerization of a mesogenic triphenylene derivative having cinnamate groups: effects of orientation and mobility of the mesogenic molecules on the photoreaction behavior'

## Beschreibung

Die Erfindung betrifft neue Triphenylenverbindungen, Verfahren zur Herstellung von diskotisch flüssigkristallinen vernetzten Polymeren unter Verwendung dieser neuen Triphenylenverbindungen sowie die Verwendung der vernetzten Polymeren als Ladungstransportmaterialien, beispielsweise in Photokopierern oder in Laserdruckern.

Photoleiterbeschichtungen auf anorganischer Basis, beispielsweise auf Basis von Selen, Tellur oder Cadmium, sowie auf Basis organischer Polymerer sind bereits bekannt. So wird beispielsweise in der EP-A-0 504 059 die Herstellung von Trommelbeschichtungen aus organischen Polymeren auf photochemischen Wege beschrieben.

Organische Photoleiter (Organic Photo Conductors, OPC) werden hauptsächlich als lichtempfindliche Materialien in der Elektrophotographie und Drucktechnik eingesetzt.

Aspekte der Wechselwirkung von Licht mit diskotisch flüssigkristallinen Systemen sind der Publikation von A.M. Fox et al. in C & EN 71 (1993), Seiten 38 bis 48 zu entnehmen.

Van der Pol et al. (Macromolecules (1990), 23, 155) beschreiben die Synthese und Eigenschaften achtfach funktionalisierter, diskotisch flüssigkristalliner Metallopthalocyanine. Thermische Vernetzung ergibt zwar unlösliche flüssigkristalline Materialien, die eine erhöhte elektrische Leitfähigkeit, besonders nach Dotieren mit Iod, zeigen. Eine Nutzung der Photoleitfähigkeit ist hier aber technisch nicht möglich, da sie von der hohen intrinsischen Leitfähigkeit überdeckt wird.

Bekannt sind auch die photoleitenden Eigenschaften niedermolekularer Flüssigkristalle in der diskotischen Phase oder einer der kalamitischen flüssigkristallinen Phasen (vgl. z.B. EP-A 254 060). Bei diesen ist jedoch die mechanische Stabilität in der flüssigkristallinen Phase nicht gewährleistet. Auch polymere diskotische flüssigkristalline Phasen, eventuell dotiert mit Trinitrofluorenon, sind bekannt und werden für die Verwendung als Photoleiter vorgeschlagen (vgl. US-A 4 865 762). Solche polymeren Systeme zeigen jedoch gravierende Mängel in der Orientierung, da es nicht möglich ist, eine defektfreie Orientierung der Kolumnen in der diskotisch flüssigkristallinen Phase zu erreichen.

Bei den derzeit praktizierten Verfahren zur Beschichtung werden zum Teil toxische Substanzen verwendet, zum Teil werden große Mengen organischer Lösungsmittel freigesetzt, die die Einhaltung spezieller Vorschriften zur Emissionsverminderung erschweren. Außerdem weisen die derzeit verwendeten organischen Photoleiter recht niedrige Ladungsträgerbeweglichkeiten auf.

Diskotisch flüssigkristalline Triphenylene zeigen ausgezeichnete Photoleitfähigkeit in der flüssigkristallinen Phase, die durch die molekulare Orientierung der Substanz verursacht wird. Dieser technische Vorteil kann jedoch nicht in der üblichen OPC-Technik verwendet werden, da dabei die flüssigkristallinen Eigenschaften verloren gehen.

Aufgabe der vorliegenden Erfindung ist es, neue polyfunktionelle Verbindungen aufzuzeigen, die sich zur Herstellung von diskotisch flüssigkristallinen vernetzten Polymeren eignen, wobei die oben geschilderten Nachteile des bekannten Standes der Technik vermieden werden.

Überraschenderweise gelingt es, durch direktes Aufbringen eines polymerisationsfahigen Gemisches aus mehrfach mit polymerisierbaren Funktionalitäten substituierten Triphenylenverbindungen und anschließender thermischer und/oder photochemischer Polymerisation die aufgebrachte Schicht zu vernetzen und zu härten. Insbesondere durch die schonende photochemische Polymerisationsweise wird die besondere Anordnung zur erhöhten Leitfähigkeit nicht zerstört.

Gegenstand der vorliegenden Erfindung sind Verbindungen der allgemeinen Formel (I) 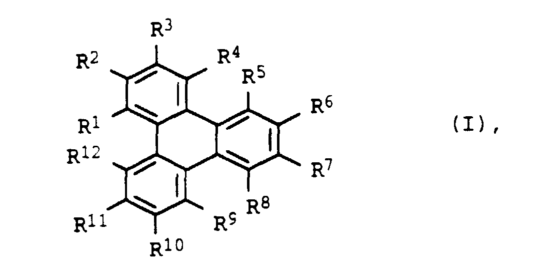 worin R¹ bis R¹² untereinander gleich oder verschieden sind und für Wasserstoff, Halogen, Alkyl, X-Alkyl mit X = 0, S oder NH oder N(Alkyl)₂, jeweils mit 1 bis 20 Kohlenstoffatomen im Alkylrest, für derartige zusätzlich substituierte Alkylreste oder für Reste, die C-C-Doppelbindungen, C-C-Dreifachbindungen, Oxiranyl- oder Thiiranylgruppen enthalten, stehen, mit der Maßgabe, daß mindestens drei der Reste R¹ bis R¹² Oxiranyl- oder Thiiranylgruppen enthaltende Reste oder C-C-Doppelbindungen enthaltende Reste der Formeln

Y-CO-CH = CH₂,

Y-CO-C(CH₃)=CH₂,

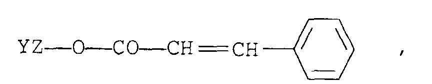

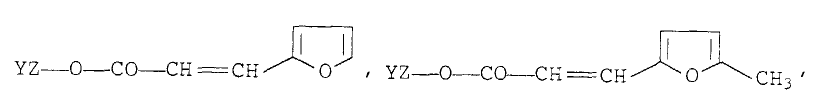

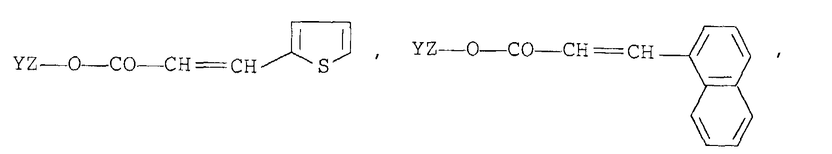

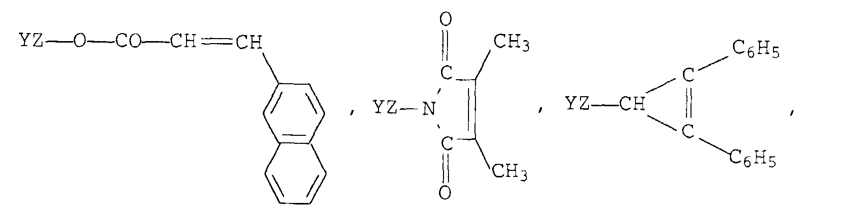

oder 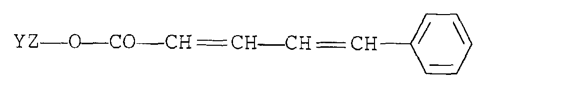 sind, worin Y für O, S oder eine Einfachbindung und Z für (CH₂)ₙ mit n = 1 bis 12 stehen.

Soweit in der Formel (I) Reste R¹ bis R¹² für zusätzlich substituierte 1 bis 20 Kohlenstoffatome enthaltende Alkylreste stehen, können sie als Substituenten COOH, OH, NCO, NH₂, NHR¹³, N(R¹³)₂ oder OR¹³ mit R¹³ = geradkettiges oder verzweigtes Alkyl mit bis zu 12 Kohlenstoffatomen, Aryl, Alkaryl, Oxaalkyl mit bis zu 4 Sauerstoffatomen und bis zu 8 Kohlenstoffatomen in der Kette oder Thiaalkyl mit bis zu 4 Schwefelatomen in der Kette enthalten.

Bevorzugt sind solche erfindungsgemäßen Verbindungen der allgemeinen Formel (I) worin drei bis sechs der Reste R¹ bis R¹² C-C-Doppelbindungen, C-C-Dreifachbindungen, Oxiranyl- oder Thiiranylgruppen enthaltende Reste sind.

Gegenstand der vorliegenden Erfindung ist auch ein Verfahren zur Herstellung von diskotisch flüssigkristallinen vernetzten Polymeren, wobei diese durch Polymerisation von Verbindungen der allgemeinen Formel (I), gegebenenfalls im Gemisch mit anderen copolymerisierbaren ethylenisch ungesättigten Verbindungen oder Oxiranyl- oder Thiiranylgruppen tragenden Verbindungen hergestellt werden.

Gegenstand der vorliegenden Erfindung ist außerdem die Verwendung der nach diesem Verfahren hergestellten diskotisch flüssigkristallinen vernetzten Polymeren als Ladungstransportmaterialien, sowie die Verwendung dieser Ladungstransportmaterialien in Photokopierern oder in Laserdruckern.

Zu den neuen Verbindungen, ihrer Herstellung sowie ihrer Weiterverarbeitung und Verwendung ist im einzelnen folgendes auszuführen.

In den neuen Verbindungen der allgemeinen Formel (I) 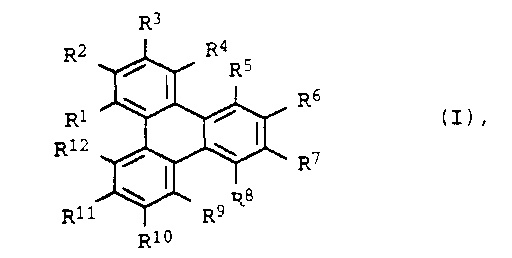 können R¹ bis R¹² untereinander gleich oder verschieden sein und für Wasserstoff, Halogen, wie z.B. F, Cl, Br, Alkyl mit 1 bis 20 Kohlenstoffatomen, wobei die Alkylreste geradkettig oder verzweigt sein können oder alicyclische Gruppen enthalten können, beispielsweise Methyl, Ethyl, n-Propyl, Isopropyl, n-Butyl, Isobutyl, tert.-Butyl, Hexyl, Cyclohexyl, Heptyl, Octyl, 2-Ethylhexyl, Lauryl, Palmityl oder Stearyl, X-Alkyl mit 1 bis 20 Kohlenstoffatomen im Alkylrest und X = O, S oder NH, beispielsweise, Methoxyl, Ethoxyl, Propoxyl, Butoxyl, Pentoxyl, Hexoxyl, Heptoxyl, Octoxyl, Nonoxyl, Decyloxy und Undecyloxy, Alkylthio, wie z.B. Methylthio bis Undecylthio, NH-Alkyl, wie z.B. Methylamino bis Undecylamino, N(Alkyl)₂, wie z.B. Dimethylamino, Diethylamino, Methylethylamino, Dipropylamino, Dibutylamino, Di-undecylamino, zusätzlich substituierte 1 bis 20 Kohlenstoffatome aufweisende Alkylreste, die als Substituenten Gruppierungen wie COOH, OH, NCO, NH₂, NHR¹³, N(R¹³)₂, OR¹³ oder COOR¹³, wobei R¹³ für ein geradkettiges oder verzweigtes Alkyl mit bis zu 10 Kohlenstoffatomen, wie z.B. Methyl, Ethyl, n-Propyl, Isopropyl, n-Butyl, Isobutyl, Pentyl oder Hexyl, für Aryl, wie z.B. Phenyl oder Biphenyl oder Terphenyl, für Alkaryl, wie z.B. Benzyl oder Phenylethyl, für Oxaalkyl mit 1 bis 4 Sauerstoffatomen und 1 bis 8 Kohlenstoffatomen, wie z.B. 1,3,6-Trioxaoctan, Thiaalkyl mit 1 bis 4 Schwefelatomen und 1 bis 8 Kohlenstoffatomen in der Kette, wobei mindestens drei der Reste R¹ bis R¹² Oxiranyl- oder Thiiranylgruppen enthaltende Reste oder C-C-Doppelbindungen enthaltende Reste der Formeln

Y-CO-CH =CH₂,

Y-CO-C(CH₃)=CH₂,

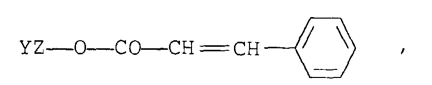

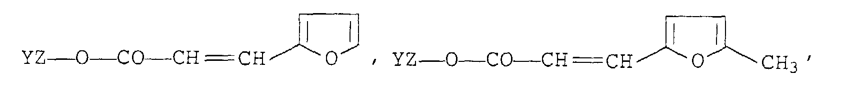

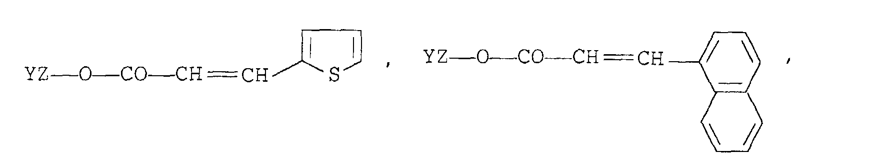

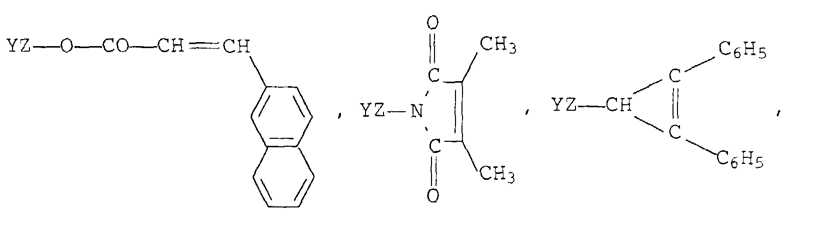

oder 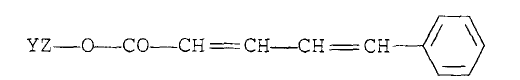 sind, worin Y für O, S oder eine Einfachbindung und Z für (CH₂)ₙ mit n = 1 bis 12 stehen.

Beispiele für derartige Reste R¹ bis R¹² sind CH₂=CH-CO-O, CH₂=C(CH₃)-CO-O, CH₂=C(CH₃)-CO-S-, 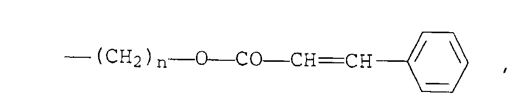 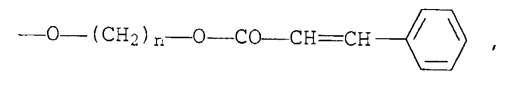 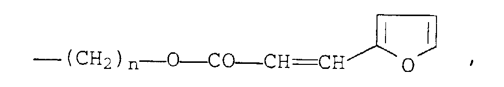 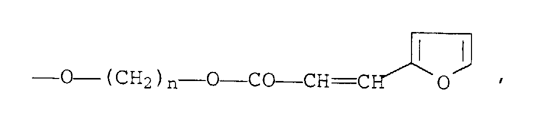 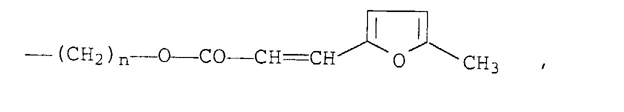 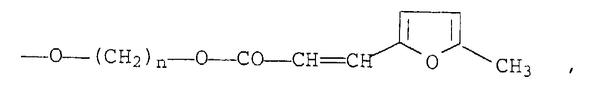 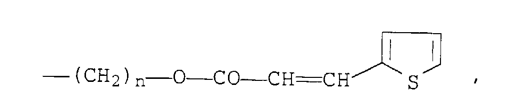 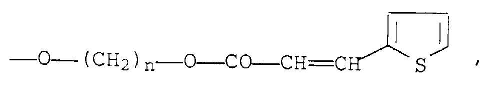 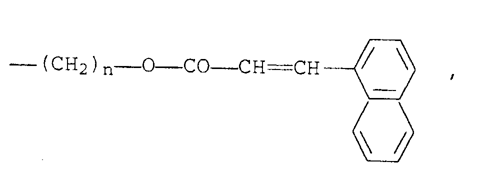 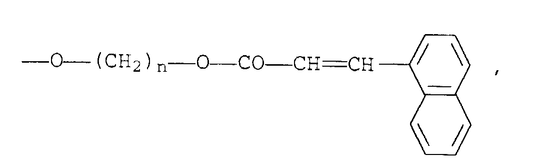 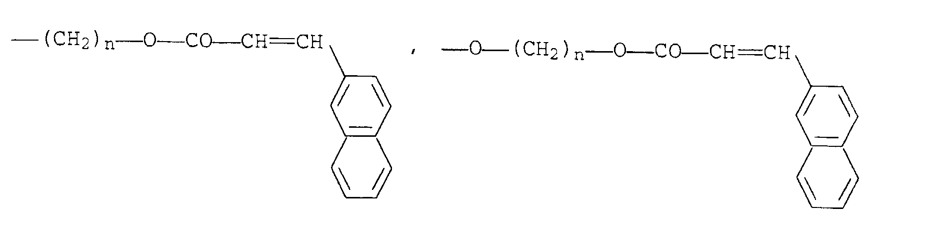 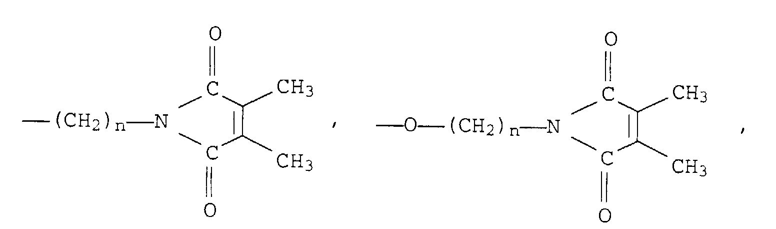 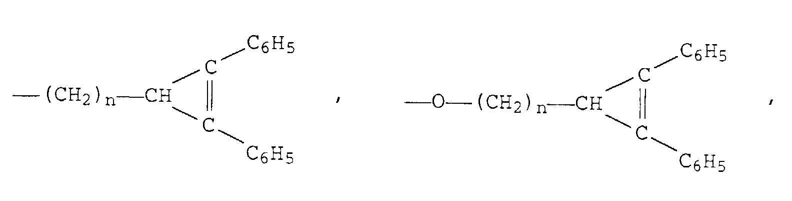 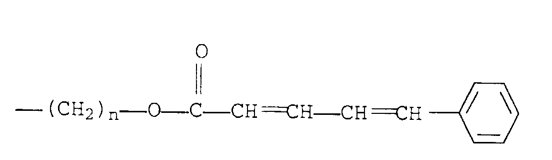 oder 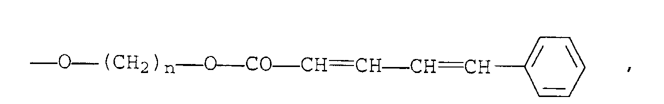 jeweils mit n = 1 bis 12, vorzugsweise 3 bis 6.

Ein besonders bevorzugter C-C-Doppelbindungen enthaltender Rest R¹ bis R¹² ist 3-Acryloyloxypropoxy.

Als C-C-Dreifachbindungen enthaltende Reste R¹ bis R¹² sind beispielsweise Ethinyl und Diacetylenyl, zu nennen.

Beispiele für Oxiranylgruppen enthaltende Reste sind

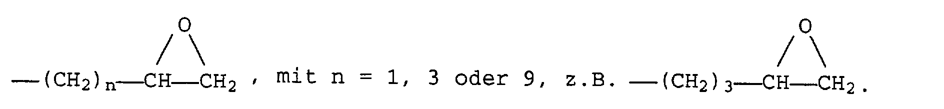

Beispiele für Thiiranylgruppen enthaltende Reste sind

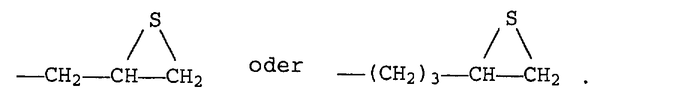

Die Herstellung der erfindungsgemäßen Verbindungen der allgemeinen Formel (I) kann nach üblichen Verfahren erfolgen, wie sie beispielsweise in I.M. Matheson, O.C. Musgrave, C.J. Webster, "Oxidation of veratrole by quinones", Chem. Commun. 1965, 278, M. Piatelli, E. Fattorusso, R.A. Nicolaus, S. Magno "The structure of melanins and melanogenesis-V", Tetrahedron 21, 3229 (1965) and K. Bechgaard, V.D. Parker, "Mono-, Di- and Trikations of Hexamethoxytriphenylene, A Novel Anodic Trimerization", J. Am. Chem. Soc. 94, 4749 (1972) beschrieben sind.

Ausgehend vom Substitutionsmuster des 2,3,6,7,10,11-Hexakis(pentyloxy)triphenylen ergeben sich prinzipiell zwei Wege, entweder eine funktionelle Gruppe für Seitengruppenpolymere, bzw. zwei oder mehr funktionelle Gruppen für Hauptkettenpolymere einzuführen:

Entweder kann man vom Hexapentoxytriphenylen eine oder mehrere der sechs Pentylreste unter Zurücklassung funktioneller Gruppen abspalten, oder es können die Pentylreste über das Hexaacetoxytriphenylen so eingeführt werden, daß nicht umgesetzte Acetoxygruppen als reaktive Stellen im Molekül zurückbleiben.

Der erste Syntheseschritt zur Funktionalisierung durch Alkylierung des Hexaacetoxytriphenylens ist der Aufbau des aromatischen Systems. Der präparativ ergiebigste Zugang zum Triphenylengerüst ist die oxidative Trimerisierung von Veratrol, die mit p-Chloranil, Eisenchlorid oder elektrochemisch erfolgen kann.

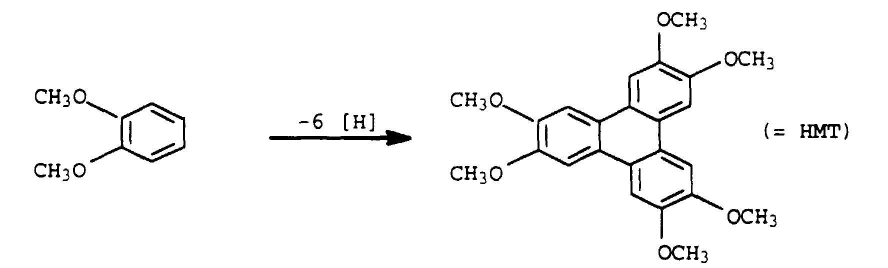

Eine vorteilhafte Modifikation der oxidativen Trimerisierung mittels p-Chloranil ist der Zuschlag von Kieselgel als Trägerstoff für das HMT, das bei der heterogenen Reaktion als feiner, kaum filtrierbarer Schaum anfällt. Dadurch kann beim Aufarbeiten rasch vom aggressiven Reaktionsmedium (Schwefelsäure/Essigsäure=1/1) auf organische Lösungsmittel gewechselt werden. Die Oxidation mit Eisenchlorid ist ebenfalls ein anwendbarer Syntheseweg.

Im zweiten Schritt können die sechs Methylethergruppen des HMT durch basenlabile Acetylschutzgruppen ersetzt werden. Die Etherspaltung kann dabei mit einem Überschuß an Bortribromid erfolgen. Das kristalline Zwischenprodukt 2,3,6,7,10,11-Triphenylenhexayltris(o-bromoborat) kann in situ mit Acetanhydrid zum 2,3,6,7,10,11-Triphenylenhexaylhexaacetat umgeestert werden.

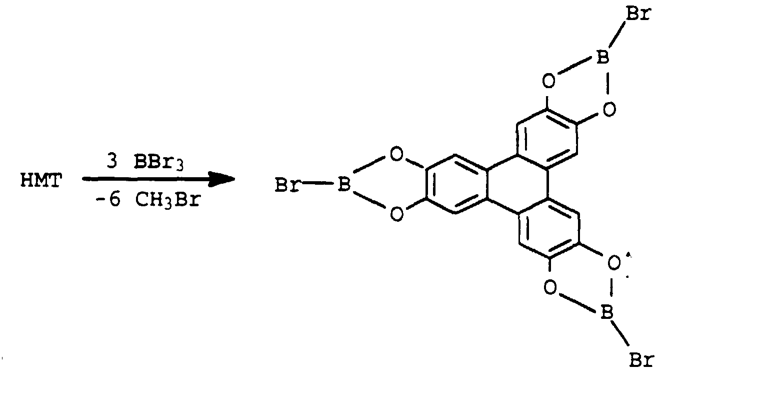

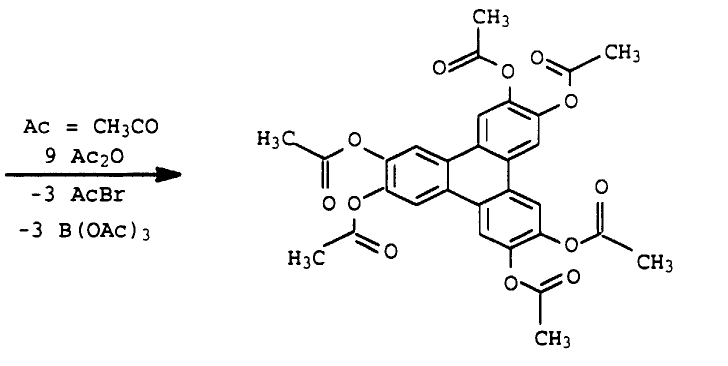

Die für die Herstellung der erfindungsgemäßen Verbindungen der allgemeinen Formel (I) geeigneten, Hydroxy- und/oder Alkoxygruppen enthaltenden Triphenylene können beispielsweise durch vollständige oder partielle Etherspaltung von 2,3,6,7,10,11-Hexalkoxytriphenylen oder durch Hydrolyse von 2,3,6,7,10,11-Triphenylenhexaylhexaacetat erhalten werden.

Die Hydroxy- und/oder Alkoxygruppen enthaltenden Triphenylene können dann wiederum beispielsweise durch Umsetzung mit Halogenalkyl(meth)acrylaten zu erfindungsgemaßen Verbindungen (I) umgesetzt werden. Alternativ können erfindungsgemaße Verbindungen (I) auch durch Umsetzung von hydroxyalkyloxysubstituiertem Triphenylen mit Alkenoylchloriden (z.B. (Meth)acrylsäurechlorid) erhalten werden. Die bei den Umsetzungen anfallenden Produktgemische können auf an sich bekannte Weise durch fraktionierte Kristallisation oder Chromatographie getrennt werden. Ferner können statistisch substituierte Triphenylene hergestellt werden durch gleichzeitige Alkylierung des 2,3,6,7,10,11.Hexahydroxytriphenylens mit Halogenalkyl(meth)acrylaten und Halogenalkylen.

Hexaalkyloxytriphenylene mit unterschiedlich langen Alkylketten im selben Molekül lassen sich chromatographisch trennen.

Bevorzugte Verbindungen der allgemeinen Formel (I) sind solche Verbindungen, bei denen mindestens drei der Reste R¹ bis R¹² C-C-Doppelbindungen aufweisen, insbesondere solche mit (Meth)acryloylgruppen wie z.B.

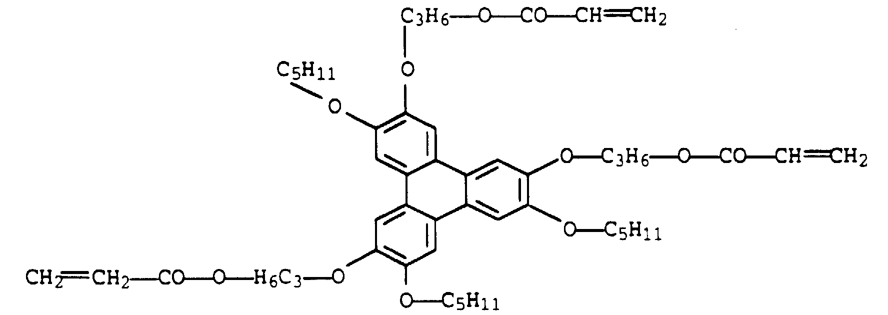

Die Herstellung von diskotisch flüssigkristallinen vernetzten Homo- oder Copolymeren unter Verwendung der erfindungsgemaßen Verbindungen der allgemeinen Formel (I) kann nach den üblichen Polymerisationsverfahren, beispielsweise thermisch in Gegenwart radikalliefernder Polymerisationsinitiatoren, wie z.B. Azobisisobutyronitril oder Benzoylperoxid, vorzugsweise durch Photopolymerisation bei Temperaturen kurz unterhalb der Klärtemperatur des Monomeren bzw. der Monomerenmischung in Gegenwart geeigneter üblicher Photoinitiatoren, wie z.B. Benzoinethern, wie z.B. Benzoinisopropylether, Benzildimethylketal, Dimethylbenzoinether, oder Acylphosphinoxidverbindungen durchgeführt werden, die beispielsweise in Mengen von 0,1 bis 10 Gew.-%, bezogen auf Verbindungen der Formel (I) eingesetzt werden können.

Es kann zweckmäßig sein, die erfindungsgemäßen mindestens trifunktionellen Verbindungen der allgemeinen Formel (I) mit bi-oder insbesondere monofunktionellen Verbindungen des Triphenylentyps, z.B. zusammen mit Pentakispentyloxy-acryloylpropyloxy-triphenylen zu copolymerisieren, wobei der Anteil an erfindungsgemäßen mindestens trifunktionellen Triphenylenverbindungen 20 bis 80 Gew.-% der Gesamtmenge des Monomerengemisches betragen sollte.

Für die Herstellung der erfindungsgemäßen diskotisch flüssigkristallinen vernetzten Polymeren und deren Weiterverwendung als Ladungstransportmaterialien in Photokopierern oder Laserdruckern ist es zweckmäßig, die Polymerisation des Monomeren bzw. des Monomerengemisches auf bestimmten Substraten, wie z.B. auf Aluminium, Stahl oder Gold durchzuführen.

Die erfindungsgemäßen diskotisch flüssigkristallinen vernetzten Polymeren sind einfach und umweltfreundlich herzustellen, da toxische Verbindungen und Abgasemission vermieden werden.

Die erfindungsgemäß hergestellten Ladungsträger zeichnen sich durch einfache Herstellbarkeit, durch Schnelligkeit des Ladungstransports, Fallenfreiheit und das Fehlen von Raumladungsbildungen aus.

### Beispiel 1

### Synthese von statistisch substituierten Tri-2'-acryloyl-ethyloxytrihexyloxytriphenylen

a) Darstellung von 2,3,6,7,10,11-Hexahydroxytriphenylen
   50,9 g Hexamethoxytriphenylen, 387 g Eisessig und 387 g 47 %ige wäßrige Bromwasserstoffsäure wurden 24 Stunden unter Stickstoff unter Rückfluß gekocht. Danach wurden der Eisessig und die Bromwasserstoffsäure zum großen Teil abdestilliert. Es fiel eine dunkle blauschwarze Substanz aus, die mit drei mal 500 ml Wasser gewaschen wird und im Vakuum getrocknet wurde. Die Ausbeute betrug 39,9 g.
b) Darstellung von statistisch substituierten Tri-2'-acryloylethyloxy-trihexyloxytriphenylen
   4,86 g (0,015 mol) 2,3,6,7,10,11-Hexahydroxytriphenylen wurden zusammen mit 8,66 g (0,0525 mol) 1-Bromhexan, 9,4 g (0,0525 mol) 2-Bromethylacrylat, 14,49 g (0,105 mol) Kaliumcarbonat und 0,1 g Phenothiazin in 50 ml Dimethylformamid gelöst bzw. aufgeschlämmt und 6 Stunden bei 80°C unter Stickstoff gerührt. Nach dem Abkühlen auf Raumtemperatur wurde auf verdünnte Salzsäure ausgefällt, das Produkt mit Methylbutylether extrahiert, die etherische Phase mit Wasser gewaschen und nach dem Trocknen mit Natriumsulfat im Vakuum eingeengt. Das erhaltene Rohprodukt wurde durch Säulenchromatographie über Kieselgel (Laufmittel: Toluol/Ethanol im Verhältnis 1:1) gereinigt und anschließend aus Ethanol umkristallisiert. Es wurden 1,8 g farblose Kristalle erhalten. Schmelzpunkt: 83-87°C
   Die Analyse mittels ¹H-NMR ergab eine durchschnittliche Substitution des Triphenylenkerns mit 2,88 Hexahexyloxyseitenketten und 3,12 2'-Acryloyl-ethyloxyseitenketten.

### Beispiel 2

### Synthese von statistisch substituierten Tri-4'-acryloyl-butyloxytrihexyloxytriphenylen

a) Darstellung von statistisch substituierten Tri-4'-acetyloxybutyloxy-trihexyloxytriphenylen
   4,86 g (0,015 mol) 2,3,6,7,10,11-Hexahydroxytriphenylen wurden zusammen mit 10,24 g (0,05255 mol) 4-Brombutylacetat, 8,66 g (0,0525 mol) 1-Bromhexan und 14,49 g (0,105 mol) Kaliumcarbonat in 50 ml Dimethylformamid gelöst bzw. aufgeschlämmt und 6 Stunden bei 80°C unter Stickstoff gerührt. Nach dem Abkühlen auf Raumtemperatur wurde auf verdünnte Salzsäure ausgefällt, das Produkt mit Methylbutylether extrahiert, die etherische Phase mit Wasser gewaschen und nach dem Trocknen mit Natriumsulfat im Vakuum eingeengt. Das erhaltene Rohprodukt wurde durch Säulenchromatographie über Kieselgel (Laufmittel: Toluol/Ethanol im Verhältnis 1:1) gereinigt. Es wurden 10,9 g farblose Kristalle erhalten.
   Die Analyse mittels ¹H-NMR ergab eine durchschnittliche Substitution des Triphenylenkerns mit 2,58 Hexahexyloxyseitenketten und 3,42 4'-Acetyloyl-butyloxyseitenketten.
b) Darstellung von statistisch substituierten Tri-4'-hydroxybutyloxy-trihexyloxytriphenylen
   4,13 g (0,0045 mol) Tri-4'-acetyloyl-butyloxy-trihexyloxytriphenylen wurden mit 3,02 g 50 %iger wäßriger Kaliumhydroxidlösung und 40 ml Ethanol versetzt und 10 Stunden unter Rückfluß gekocht. Nach dem Abkühlen auf Raumtemperatur wurde die Mischung auf Eiswasser gegeben, das ausgefallene Produkt mit Methylbutylether extrahiert, die etherische Phase mit Wasser gewaschen nach dem Trocknen mit Natriumsulfat im Vakuum eingeengt. Das erhaltene Rohprodukt wurde durch Säulenchromatographie über Kieselgel (Laufmittel: Toluol/Ethanol im Verhältnis 4:1) gereinigt. Es wurde 2,2 g farblose Kristalle erhalten.
   Die Analyse mittels ¹-H-NMR ergab eine durchschnittliche Substitution des Triphenylenkerns mit 3 Hexahexyloxyseitenketten und 3 4'-Hydroxybutyloxyseitenketten.
c) Darstellung von statistisch substituierten Tri-4'-acryloylbutyloxy-trihexyloxytriphenylen
   1,85 g (0,0023 mol) Tri-4'-hydroxy-butyloxy-trihexyloxytriphenylen werden mit 6,7 g (0,093 mol) Acrylsäure, 0,1 g 2,6-Di-tert.-butyl-p-Kresol, 0,2 g p-Toluolsulfonsäure und 50 ml 1,1,2-Trichlorethan versetzt und 24 Stunden unter Rühren am Wasserabscheider gekocht. Nach dem Abkühlen auf Raumtemperatur wurde mit Wasser und Natriumhydrogencarbonatlösung extrahiert und die organische Phase nach dem Trocknen mit Natriumsulfat im Vakuum eingeengt. Das erhaltene Rohprodukt wurde durch Säulenchromatographie über Kieselgel (Laufmittel: Toluol/Ethanol im Verhältnis 1:1) gereinigt. Zum Produkt wurde Ethanol gegeben und die Ethanolphase bei -30°C abdigeriert. Es wurden 1,35 g farbloses Öl erhalten. Die Analyse mittels ¹H-NMR ergab eine durchschnittliche Substitution Triphenylenkerns mit 3 Hexahexyloxyseitenketten und 3,4'-Acryloyl-butyloxyseitenketten.

### Beispiel 3

### Herstellung von 2,6,10-Tri-3'-acryloyl-propyloxy-3,7,11-tripentyloxytriphenylen und 2,6,11-Tri-3'-acryloyl-propyloxy3,7,10-tripentyloxytriphenylen (Variante 1)

a) 2,6,10-Trihydroxy-3,7,11-tripentyloxytriphenylen und 2,6,11-Trihydroxy-3,7,10-tripentyloxytriphenylen
   Ausgehend vom 2,3,6,7,10,11-Hexapentyloxytriphenylen, das durch Alkylierung von 2,3,6,7,10,11-Hexahydroxy- bzw. 2,3,6,7,10,11-Hexaacetoxytriphenylen nach bekannten Verfahren hergestellt werden konnte, wurden die beiden Isomere 2,6,10-Trihydroxy-3,7,11-tripentyloxytriphenylen und 2,6,11-Trihydroxy-3,7,10-tripentyloxytriphenylen durch dreifache Etherspaltung mit 9-Brom-9-borabicyclo[3,3,1]nonan in Methylenchlorid hergestellt.
   In einem mit Argon gespülten Dreihalskolben wurden hierzu 3,0 g 2,3,6,7,10,11-Hexapentyloxytriphenylen in 30 ml Dichlormethan gelöst und langsam 18,1 ml einer 1-molaren 9-Brom-9-borabicyclo[3,3,1]nonanlösung in Methylenchlorid zugetropft. Die Reaktionsmischung wurde anschließend 30 Stunden bei Raumtemperatur gerührt, bevor 1,1 ml Ethanolamin vorsichtig zugegeben wurden. Nach einigen Minuten wurden weiteres Dichlormethan und Wasser zugegeben und das Produkt ausgeschüttelt. Die produkthaltige Dichlormethanphase wurde mit Magnesiumsulfat getrocknet und das Dichlormethan abdestilliert. Gereinigt und getrennt wurden die beiden Isomere durch eine säulenchromatographische Reinigung mit dem Laufmittel Dichlormethan/Hexan im Verhältnis 3:2 und reinem Dichlormethan. Insgesamt wurden zwei weiße Pulver in einer Ausbeute von 1,82 g (80,7 % der Theorie) erhalten. Die Ausbeute an symmetrischen Produkt (2,6,10-Trihydroxy-3,7,11-tripentyloxytriphenylen) betrug 0,81 g (35,9 %) und die Ausbeute am unsymmetrischen Produkt (2,6,11-Trihydroxy-3,7,10-tripentyloxytriphenylen) betrug 1,01 g (44,8 %). Beide Isomere wurden mittels NMR- und IR-Spektroskopie charakterisiert.
   2,6,10-Trihydroxy-3,7,11-tripentyloxytriphenylen: k₁ 120,5°C k₂ 140°C i
   2,6,11-Trihydroxy-3,7,10-tripentyloxytriphenylen: k 146°C i
b) 2,6,10-Tri-3'-hydroxypropyloxy-3,7,11-tripentyloxytriphenylen und 2,6,11-Tri-3'-hydroxypropyloxy-3,7,10-tripentyloxytriphenylen
   700 mg (1,31 mmol) 2,6,11-Trihydroxy-3,7,10-tripentyloxytriphenylen und 0,819 g (5,89 mmol) Brompropanol wurden in 10 ml 2-Pentanon gelöst. Nach Zugabe von 5,5 g Kaliumcarbonat und 40 mg Kaliumiodid erhitzte man für 6 Stunden auf 80°C (Ölbadtemperatur). Dann wurde die Reaktionsmischung zum Abtrennen der unlöslichen anorganischen Bestandteile filtriert und mit Aceton gewaschen. Abdestillieren der Lösungsmittel und anschließende Chromatographie über Kieselgel mit dem Laufmittel Dichlormethan/Essigester im Verhältnis 1 1:1,2 ergab 412 mg (44,4 % d.Th.) weißes Produkt.
   R_{f}(CH₂Cl₂/MeOH: 10/1) = 0,50; Phasenverhalten: k 86 296 Dₕ 110 i:
   EA: C₄₂H₆₀O₉ (M_{w} = 708,93): Ber. C: 71,16 %, H: 8,53 %, gef.
   C: 70,46 %, H: 8,43 %, ¹H-NMR (200 MHz, CDCl₃): δ (ppm) = 8,0-7,73 (m, 6H, H_{arom.}), 4,41 (t, 6H, OCH₂CH₂CH₂OH, J = 5,7 Hz), 4,23-4,15 (m, 6H, OCH₂(CH₂)₃CH₃), 3,96 (t, 6H, OCH₂CH₂CH₂OH, J = 4,9 Hz), 2,95 (s, 3H, OH), 2,23-2,15 (m, 6H, OCH₂CH₂CH₂OH), 2,00-1,87 (m, 6H, OCH₂CH₂(CH₂)₂CH₃), 1,58-1,34 (m, 12H, OCH₂CH₂(CH₂)₂CH₃), 0,95 (t, 15H, CH₃, J = 6,9 Hz); FD-MS: m/z: 708,4 [M]·⁺.
   Analog dieser Vorschrift wurde auch das symmetrisch substituierte 2,6,10-Tri-3'-hydroxy-propyloxy-3,7,11-tripentyloxytriphenylen in 40 %iger Ausbeute erhalten.
c) 2,6,10-Tri-3'-acryloyl-propyloxy-3,7,11-tripentyloxytriphenylen und 2,6,11-Tri-3'-acryloyl-propyloxy-3,7,10-tripentyloxytriphenylen
   500 mg (0,71 mmol) 2,6,11-Tri-3'-hydroxypropyloxy-3,7,10-tripentyloxytriphenylen, 1,5 ml Triethylamin und eine Spatelspitze 2,6-Di-tert.butyl-p-Kresol wurden in 5 ml Dichlormethan gelöst. Anschließend wurden unter Eiskühlung und Argonatmosphäre 210 mg (2,33 mmol) Acrylsäurechlorid (gelöst in 3 ml Dichlormethan) zugetropft. Es wurde langsam auf Raumtemperatur erwärmt und weitere 20 Stunden gerührt. Danach verdünnte man mit 20 ml Dichlormethan und extrahierte mit 1-normaler Natriumhydrogencarbonatlösung und Wasser. Nach Abziehen des Lösungsmittels erfolgte eine chromatographische Reinigung über Kieselgel mit dem Laufmittel Dichlormethan/ Methanol im Verhältnis 60:1 und daran anschließend wurde aus Ethanol umkristallisiert. Die Ausbeute an weißem 2,6,11-Tri-3'-acryloyl-propyloxy-3,7,10-tripentyloxytriphenylen betrug 129,4 mg (21,1 % d. Th.).
   R_{f}(CH₂Cl₂/MeoH 300:1)=0,34; Smp.: 68°C;
   EA.:C₅₁H₆₆O₁₂ (M_{w}=871,081); Ber. C: 70,32 %, H: 7,64 %, gef. C: 69,92 %, H: 7,23 %;
   ¹H-NMR (200 MHz, CDCl₃) ; δ (ppm)=7,84 + 7,81 (2 x s, 6H, H_{arom.}), 6,41 (dd, 3H, COCH=CH₂) Jₜᵣₐₙₛ=17,3 Hz, J_{gem}=1,5 Hz), 6,13 (dd, 3H, COCH=CH₂, J_{cis}=10,3 Hz, Jₜᵣₐₙₛ=17,2 Hz), 5,81 (dd, 3H, COCH=CH₂, J_{cis}=10,3, J_{gem}=1,5 Hz), 4,47 (t, 6H, OCH₂(CH₂)₂OCO, J=6,3 Hz), 4,32 (t, 6H, O(CH₂)₂CH₂OCO, J=6, 1 Hz), 4,21 (t, 6H, OCH₂(CH₂)₃CH₃, J=6,6 Hz), 2,36-2,23 (m, 6H, OCH₂CH₂CH₂O), 2,00-1,86 (m, 6H, OCH₂CH₂(CH₂)₂CH₃), 1,61-1,34 (m, 12H, OCH₂CH₂(CH₂)₂CH₃), 0,95 (t, 9H, CH₂CH₃, J=6,9 Hz);
   FD-MS: m/z: 870,5[M]·⁺
   Analog dieser Vorschrift wurde auch das symmetrisch substituierte 2,6,10-Tri-3'-acryloylpropyloxy-3,7,11-tripentyloxytriphenylen in 26 %iger Ausbeute erhalten.

### Beispiel 4

### 2,6,10-Tri-3'-acryloyl-propyloxy-3,7,11-tripentyloxytriphenylen und 2,6,11-Tri-3'-acryloyl-propyloxy-3,7,10-tripentyloxytriphenylen (Variante 2)

a) 1-Hydroxy-2-pentyloxybenzol
   137,64 g (1,25 mol) Brenzcatechin und 188,63 g (1,25 mol) 1-Brompentan wurden nach Zugabe von 95,1 g (0,69 mol) Kaliumcarbonat und 321,2 g Methyl-isobutylketon 23 Stunden unter Rückfluß gekocht. Nach dem Abkühlen wurde das Kaliumcarbonat abfiltriert und das Methyl-isobutylketon unter Vakuum abdestilliert. Anschließend wurde der ölige Rückstand im Ölpumpenvakuum über eine kurze Kolonne fraktioniert destilliert. Dabei wurden bei 5 mbar und einer Siedetemperatur von 88-92°C 127,3 g 1-Hydroxy-2-pentyloxybenzol erhalten.
b) 1-3'-Hydroxy-propyloxy-2-pentyloxybenzol
   124 g (0,69 mol) 1-Hydroxy-2-pentyloxybenzol und 100,8 g (0,69 mol) wurden nach Zugabe von 50,1 g (0,36 mol) Kaliumcarbonat und 115,6 g Methyl-isobutylketon 40 Stunden unter Rückfluß gekocht. Nach dem Abkühlen wurde das Kaliumcarbonat abfiltriert und das Methylisobutylketon unter Vakuum abdestilliert. Anschließend wurde der ölige Rückstand im Rückstand im Ölpumpenvakuum über eine kurze Kolonne fraktioniert destilliert. Dabei wurden bei 5 mbar und einer Siedetemperatur von 140-148°C 78,2 g 1-3'-Hydroxy-propyloxy-2-pentyloxybenzol erhalten.
c) 2,6,10-Tri-3'-hydroxypropyloxy-3,7,11-tripentyloxytriphenylen und 2,6,11-Tri-3'-hydroxypropyloxy-3,7,10-tripentyloxytriphenylen
   1 g (4,2 mmol) 1-3 -Hydroxy-propyloxy-2-pentyloxybenzol wurde in 5 ml Dichlormethan unter Argonatmosphäre gelöst. Nach der portionsweisen Zugabe von 1,59 g (9,8 mmol) Eisen-(III)-chlorid wurde mit 3 Tropfen konzentrierter Schwefelsäure versetzt und auf 50°C Ölbadtemperatur erhitzt. Die Reaktionsmischung wurde nach 2 Stunden abgekühlt und die Reaktion durch Zugabe von 5 ml Methanol beendet. Das Lösungsmittel wurde abdestilliert und das Produkt mittels zweifacher Chromatographie gereinigt. Das Laufmittel war beim ersten Mal Dichlormethan/Methanol im Verhältnis 30:1 und beim zweiten Mal Dichlormethan/Methanol im Verhältnis 40:1. Dabei konnte das symmetrische 2,6,10-Tri-3'-hydroxypropyloxy-3,7,11-tripentyloxytriphenylen vollständig von dem unsymmetrischen 2,6,11-Tri-3'-hydroxypropyloxy-3,7,10-tripentyloxytriphenylen getrennt werden. Die Ausbeute betrug 543 mg für das unsymmetrische Produkt und 80 mg für das symmetrische Produkt.
   Die anschließende Veresterung mit Acrylsäure wurde analog Beispiel 3 durchgeführt.

### Beispiel 5

Eine Mischung aus Pentakispentyloxyacryloylpropyloxy-triphenylen (monofunktionell, flüssigkristallin) und Trispentyloxytrisacryloylpropyloxy-triphenylen (trifunktionell, nicht flüssigkristallin) wurde im Verhältnis 4:1 miteinander verschmolzen. Die Mischung wies diskotisch flüssigkristalline Eigenschaften auf. Nach Zusatz eines radikalischen Photoinitiators (1 mol-% Benzildimethylketal, z.B. Darocur® 1664 der Fa. CIBA-GEIGY AG) wurde die Mischung durch Bestrahlung mit UV-Licht einer Quecksilber-Mitteldruck- oder Hochdrucklampe bei einer Temperatur kurz unterhalb der Klärtemperatur des Monomerengemisches - also in der flüssigkristallinen Phase - vernetzend polymerisiert. Da die Orientierung der Moleküle in der Probe durch die Photopolymerisation nicht gestört wird, beobachtet man sowohl vor als auch nach der Polymerisation Photoleitung. Auch durch Aufheizen über den Klärpunkt des flüssigkristallinen vernetzten Copolymerisates und anschließendes Abkühlen ließ sich diese Orientierung nicht zerstören. Zusätzlich zeigte das Copolymerisat hervorragende Oberflächenbeschaffenheit und sehr gute mechanische Eigenschaften.

### Vergleichsbeispiel

Eine Mischung aus 99 mol-% Pentakispentyloxy-acryloylpropyloxytriphenylen (monofunktionell, flüssigkristallin) und 1mol-% eines Photoinitiators (z.B. Benzildimethylketal wie Darocur® 1664) wurde im Isotropen bei 98°C verschmolzen. Die Mischung wies diskotisch flüssigkristallines Verhalten auf. Durch Bestrahlung mit UV-Licht wurde die durch Abkühlen aus dem Isotropen hergestellte orientierte Phase polymerisiert.

Beim Abkühlen dieses Polymerisationsansatzes auf Raumtemperatur entmischte er sich in polymere und kristalline niedermolekulare Bereiche, die durch ihre ungenügende Orientierung den Photostrom nicht mehr leiteten.

## Patentansprüche

1. Verbindungen der allgemeinen Formel (I) 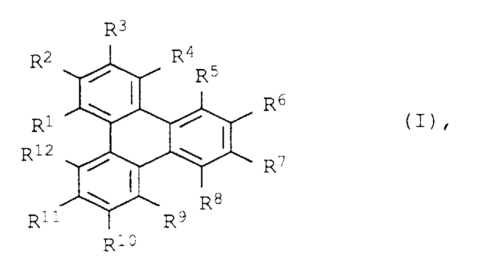 worin R¹ bis R¹² untereinander gleich oder verschieden sind und für Wasserstoff, Halogen, Alkyl, X-Alkyl mit X = 0, S oder NH oder N(Alkyl)₂, jeweils mit 1 bis 20 Kohlenstoffatomen im Alkylrest, für derartige zusätzlich substituierte Alkylreste oder für Reste, die C-C-Doppelbindungen, C-C-Dreifachbindungen, Oxiranyl- oder Thiiranylgruppen enthalten, stehen, mit der Maßgabe, daß mindestens drei der Reste R¹ bis R¹² Oxiranyl- oder Thiiranylgruppen enthaltende Reste oder
C-C-Doppelbindungen enthaltende Reste der Formeln
Y-CO-CH=CH₂,
Y-CO-C(CH₃)=CH₂, 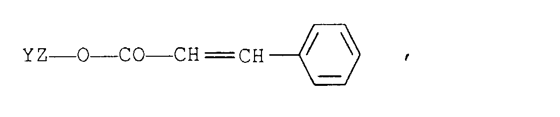 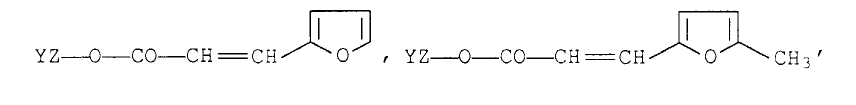 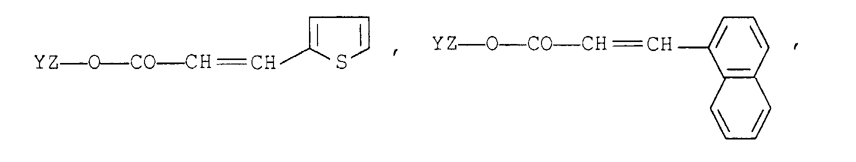 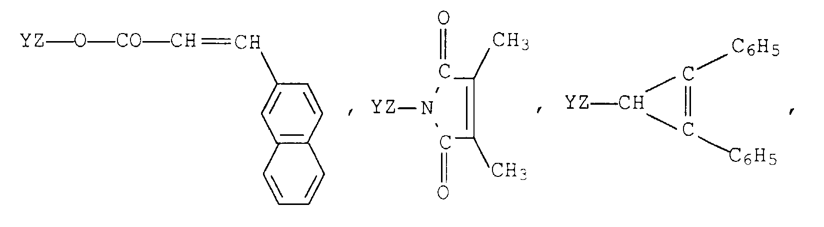
oder 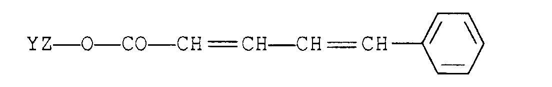 sind, worin Y für O, S oder eine Einfachbindung und Z für (CH₂)ₙ mit n = 1 bis 12 stehen.

2. Verbindungen nach Anspruch 1, dadurch gekennzeichnet, daß die zusätzlich substituierten 1 bis 20 Kohlenstoffatome enthaltenden Alkylreste als Substituenten COOH, OH, NCO, NH₂, NHR¹³, N(R¹³)₂ oder OR¹³ mit R¹³ = geradkettiges oder verzweigtes Alkyl mit bis zu 12 Kohlenstoffatomen, Aryl, Alkaryl, Oxaalkyl mit bis zu 4 Sauerstoffatomen in der Kette oder Thiaalkyl mit bis zu 4 Schwefelatomen in der Kette enthalten.

3. Verbindungen der allgemeinen Formel (I) nach Anspruch 1, dadurch gekennzeichnet, daß drei bis sechs der Reste R¹ bis R¹² C-C-Doppelbindungen, C-C-Dreifachbindungen, Oxiranyl- oder Thiiranylgruppen enthaltende Reste sind.

4. Verfahren zur Herstellung von diskotisch flüssigkristallinen vernetzten Polymeren, dadurch gekennzeichnet, daß diese durch Polymerisation von Verbindungen der allgemeinen Formel (I) gemäß Anspruch 1, gegebenenfalls im Gemisch mit anderen copolymerisierbaren ethylenisch ungesättigten Verbindungen oder Oxiranyl- oder Thiiranylgruppen tragenden Verbindungen hergestellt werden.

5. Verwendung der nach dem Verfahren gemäß Anspruch 4 hergestellten diskotisch flüssigkristallinen vernetzten Polymeren als Ladungstransportmaterialien.

6. Verwendung der Ladungstransportmaterialien nach Anspruch 5 in Photokopierern oder in Laserdruckern.

## Claims

1. A compound of the formula (I) 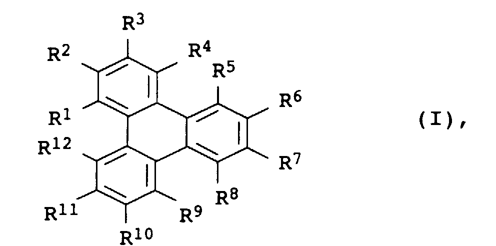 where R¹ to R¹² are identical or different and are each hydrogen, halogen, alkyl, X-alkyl, where X is O, S or NH, or N(alkyl)₂, where each alkyl radical is of 1 to 20 carbon atoms, or are each such additionally substituted alkyl radicals or are each radicals which contain C-C double bonds, C-C triple bonds, oxiranyl groups or thiiranyl groups, with the proviso that at least three of the radicals R¹ to R¹² are radicals containing oxiranyl or thiiranyl groups or radicals containing C-C double bonds, of the formulae
Y-CO-CH-CH₂,
Y-CO-C(CH₃) = CH₂, 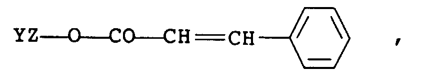 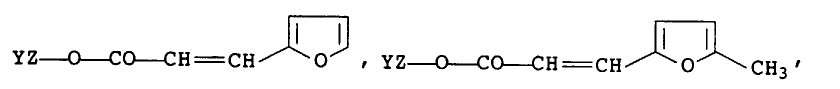 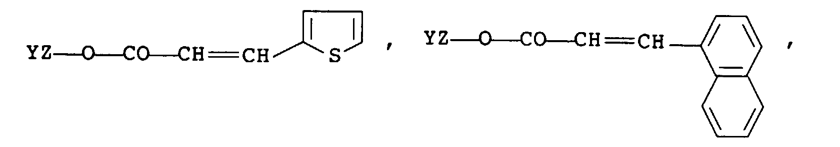 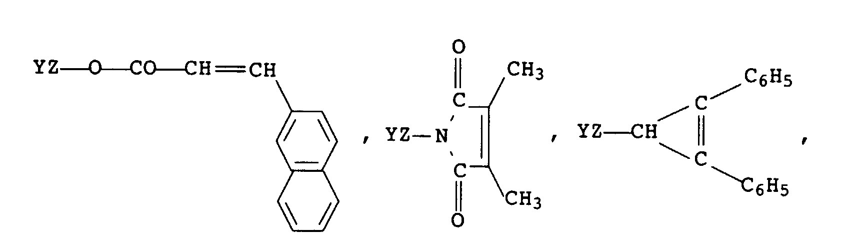
or 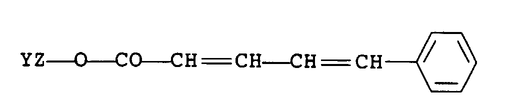 where Y is O, S or a single bond, Z is (CH₂)ₙ and n is from 1 to 12.

2. A compound as claimed in claim 1, wherein the additionally substituted alkyl radicals of 1 to 20 carbon atoms contain, as substituents, COOH, OH, NCO, NH₂, NHR¹³, N(R¹³)₂ or OR¹³, where R¹³ is straight-chain or branched alkyl of up to 12 carbon atoms, aryl, alkaryl, oxaalkyl having up to 4 oxygen atoms in the chain or thiaalkyl having up to 4 sulfur atoms in the chain.

3. A compound of the formula (I) as claimed in claim 1, wherein from three to six of the radicals R¹ to R¹² are radicals containing C-C double bonds, C-C triple bonds or oxiranyl or thiiranyl groups.

4. A process for the preparation of a discotic liquid crystalline crosslinked polymer, wherein said polymer is prepared by polymerization of compounds of the formula (I) as claimed in claim 1, if necessary as a mixture with other copolymerizable ethylenically unsaturated compounds or compounds carrying oxiranyl or thiiranyl groups.

5. Use of a discotic liquid crystalline crosslinked polymer prepared by a process as claimed in claim 4 as a charge transport material.

6. Use of the charge transport material as claimed in claim 5 in photocopiers or in laser printers.

## Revendications

1. Composés de formule générale (I) 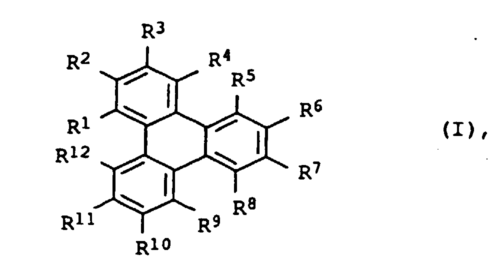 dans laquelle R¹ à R¹² sont identiques les uns aux autres ou différents les uns des autres et représentent des atomes d'hydrogène, des atomes d'halogène, des groupes alkyle, X-alkyle où X = O, S ou NH, ou N(alkyle)₂, chacun ayant de 1 à 20 atomes de carbone dans le fragment alkyle, des radicaux alkyle de ce type, présentant des substitutions supplémentaires, ou encore des radicaux qui contiennent des doubles liaisons C-C, des triples liaisons C-C, des groupes oxirannyle ou thiirannyle, à la condition qu'au moins trois des radicaux R¹ à R¹² soient des radicaux contenant des groupes oxirannyle ou thiirannyle, ou encore des radicaux contenant des doubles liaisons C-C ayant les formules suivantes :
Y-CO-CH= CH₂,
Y-CO-C(CH₃)=CH₂, 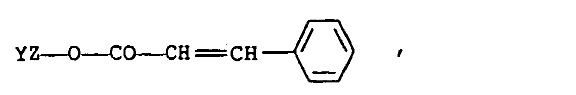 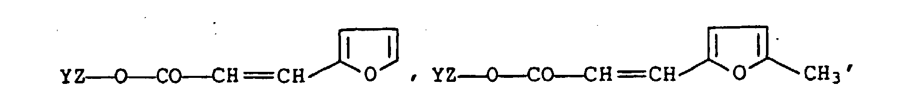 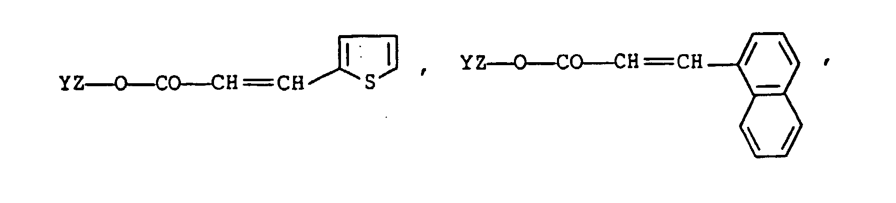 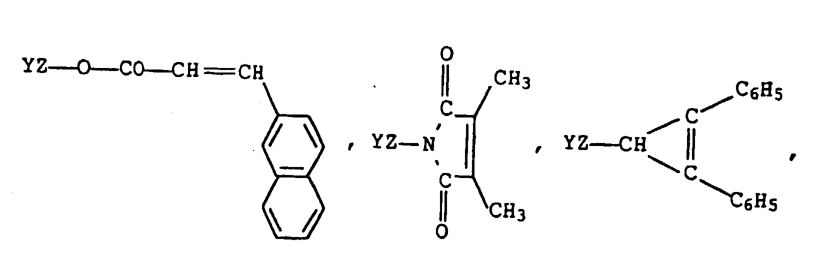
ou 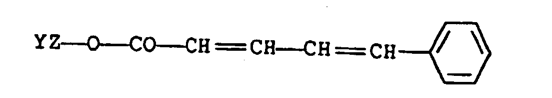 où Y est O, S ou une liaison simple, et Z représente (CH₂)ₙ, où n = 1 à 12.

2. Composés selon la revendication 1, caractérisés en ce que les radicaux alkyle portant une substitution supplémentaire et contenant de 1 à 20 atomes de carbone contiennent en tant que substituants COOH, OH, NCO, NH₂, NHR¹³, N(R¹³)₂ ou OR¹³, où R¹³ est un groupe alkyle à chaîne droite ou ramifiée ayant jusqu'à 12 atomes de carbone, aryle, alcaryle, oxaalkyle ayant jusqu'à 4 atomes d'oxygène dans la chaîne, ou thiaalkyle ayant jusqu'à 4 atomes de soufre dans la chaîne.

3. Composés de formule générale (I) selon la revendication 1, caractérisé en ce que trois à six des radicaux R¹ à R¹² sont des radicaux contenant des doubles liaisons C-C, des triples liaisons C-C, des groupes oxirannyle ou thiirannyle.

4. Procédé de préparation de polymères réticulés cristaux liquides discotiques, caractérisé en ce que ces derniers sont préparés par copolymérisation de composés de formule générale (I) selon la revendication 1, éventuellement en mélange avec d'autres composés polymérisables à insaturation éthylénique ou composés portant des groupes oxirannyle ou thiirannyle.

5. Utilisation des polymères réticulés cristaux liquides discotiques préparés selon la revendication 4, en tant que matériaux de transport de charge.

6. Utilisation des matériaux de transport de charge selon la revendication 5 dans des photocopieurs ou des imprimantes laser.
